# EUROPEAN PATENT APPLICATION

(11) **EP 3 885 451 A1**
(43) Date of publication of application: **29.09.2021**
(21) Application number: 21163170.0
(22) Date of filing: 17.03.2021
(51) Int. Cl.: C12Q 1/6851

(54) **METHOD FOR DETERMINING SUCCESS OR FAILURE OF NUCLEIC ACID AMPLIFICATION, DEVICE FOR DETERMINING SUCCESS OR FAILURE OF NUCLEIC ACID AMPLIFICATION, AND NUCLEIC ACID AMPLIFICATION ANALYSIS SYSTEM**

(30) Priority: 27.03.2020 JP 2020058282
(71) Applicant: SYSMEX CORPORATION, Kobe-shi Hyogo 651-0073 (JP)
(72) Inventor: Asao, Kazuki, Hyogo, 651-0073 (JP); Yamaguchi, Kosuke, Hyogo, 651-0073 (JP)
(74) Representative: Hoffmann Eitle

(57) **Abstract**

PROBLEM: To provide a technique for determining the success or failure of nucleic acid amplification, which makes it possible to easily determine the success or failure of the nucleic acid amplification reaction.

SOLUTION: The method for determining the success or failure of a nucleic acid amplification according to one aspect of the present invention includes obtaining a measurement value corresponding to the amount of nucleic acid amplified by the nucleic acid amplification reaction of the measurement sample containing nucleic acid in a state in which the nucleic acid amplification reaction has reached a saturated state, and determining the success or failure of the nucleic acid amplification reaction based on the acquired measurement value.

## Description

### CROSS REFERENCE TO RELATED APPLICATIONS

This application claims priority from prior Japanese Patent Application Publication No. 2020-058282, files on March 27, 2020 entitled "METHOD FOR DETERMINING SUCCESS OR FAILURE OF NUCLEIC ACID AMPLIFICATION, DEVICE FOR DETERMINING SUCCESS OR FAILURE OF NUCLEIC ACID AMPLIFICATION, AND SYSTEM FOR DETERMINING SUCCESS OR FAILURE OF NUCLEIC ACID AMPLIFICATION", the entire contents of which are incorporated herein by reference.

### FIELD OF THE INVENTION

The present invention relates to a method for determining success or failure of nucleic acid amplification, a device for determining success or failure of nucleic acid amplification, and a nucleic acid amplification analysis system.

### BACKGROUND

Techniques for analyzing nucleic acid amplification are known in which, with regard to the sample to be measured, a nucleic acid amplification reaction curve is used in which the first axis of a two-dimensional graph shows the measurement value for the nucleic acid amplification product and the second axis of the two-dimensional graph shows the value indicating the progress of the nucleic acid amplification reaction. In this type of nucleic acid amplification analysis, to ensure the reliability of the analysis result it is necessary to determine the success or failure of the amplification reaction of the nucleic acid contained in the sample. For example, Japanese Unexamined Patent Application Publication No. 2015-225032 discloses a nucleic acid amplification analysis device that obtains beforehand a normal nucleic acid amplification reaction curve by measuring a calibrator, a positive control or the like, then obtains a nucleic acid amplification reaction curve by measuring a sample, and determines the success or failure of the amplification reaction of the nucleic acid contained in the sample based on the result of comparison with the acquired normal nucleic acid amplification reaction curve.

### PRIOR ART DOCUMENT

### Patent Document

Japanese Unexamined Patent Application Publication No. 2015-225032.

### SUMMARY OF THE INVENTION

### PROBLEM TO BE SOLVED BY THE INVENTION

In the conventional nucleic acid amplification analyzer described in Japanese Unexamined Patent Application Publication No. 2015-225032, it is necessary to measure a calibrator, a positive control and the like in advance, and register the obtained results. Since this measurement and registration are required every time the reagent is exchanged, advance preparation for determining the success or failure of the nucleic acid amplification reaction becomes complicated.

Therefore, the present invention provides a technique for determining the success or failure of nucleic acid amplification, which makes it possible to easily determine the success or failure of the nucleic acid amplification reaction.

### MEANS FOR SOLVING THE PROBLEM

The method for determining the success or failure of a nucleic acid amplification according to one aspect of the present invention includes obtaining a measurement value corresponding to the amount of nucleic acid amplified by the nucleic acid amplification reaction of the measurement sample containing nucleic acid in a state in which the nucleic acid amplification reaction has reached a saturated state, and determining the success or failure of the nucleic acid amplification reaction based on the acquired measurement value.

The method for determining the success or failure of the nucleic acid amplification according to this aspect of the present invention obtains a measurement value corresponding to the amount of nucleic acid amplified by the nucleic acid amplification reaction of the measurement sample containing the nucleic acid in a state in which the nucleic acid amplification reaction has reached a saturated state, and determines the success or failure of the nucleic acid amplification reaction based on the acquired measurement value. Therefore, it is possible to easily determine the success or failure of the nucleic acid amplification reaction.

The device for determining the success or failure of nucleic acid amplification according to one aspect of the present invention includes a nucleic acid amplification unit that amplifies the nucleic acid contained in the measurement sample, a detection unit that detects nucleic acid amplification by the nucleic acid amplification unit and outputs a measurement value, and a control unit configured to control the nucleic acid amplification unit and the detection unit, obtain the measurement value corresponding to the amplified amount of the nucleic acid in a state in which the nucleic acid amplification reaction has reached the saturation state based on the output from the detection unit, and determine the success or failure of the nucleic acid amplification reaction based on the obtained measurement value.

The method for determining the success or failure of nucleic acid amplification according to this aspect of the present invention obtains a measurement value corresponding to the amount of nucleic acid amplified by the nucleic acid amplification reaction of the measurement sample containing nucleic acid in a state in which the nucleic acid amplification reaction has reached a saturation state, and determines the success or failure of the nucleic acid amplification reaction based on the acquired measurement value. Therefore, it is possible to easily determine the success or failure of the nucleic acid amplification reaction.

The system for determining nucleic acid amplification of one aspect of the present invention includes a nucleic acid amplification analyzer having a nucleic acid amplification unit that amplifies nucleic acid contained in a measurement sample, and a detection unit that detects nucleic acid amplification by the nucleic acid amplification unit and outputs a measurement value, and a determination unit, communicably connected to the nucleic acid amplification analyzer through a network, configured to obtain the measurement value corresponding to the amplified amount of the nucleic acid in a state in which the nucleic acid amplification reaction has reached the saturation state based on the output from the detection unit, and determine the success or failure of the nucleic acid amplification reaction based on the obtained measurement value.

According to the this aspect, the system for determining the success or failure for nucleic acid amplification obtains a measurement value corresponding to the amount of nucleic acid amplified by the nucleic acid amplification reaction of the measurement sample containing nucleic acid in a state where the nucleic acid amplification reaction has reached a saturation state, and determines the success or failure of the nucleic acid amplification reaction based on the obtained measurement values. Therefore, it is possible to easily determine the success or failure of the nucleic acid amplification reaction.

### EFFECTS OF THE INVENTION

The present invention provides a technique for determining the success or failure of nucleic acid amplification, which makes it possible to easily determine the success or failure of the nucleic acid amplification reaction.

### BRIEF DESCRIPTION OF THE DRAWINGS

FIG. 1 is a diagram showing an example of the structure of a nucleic acid amplification analysis system according to one embodiment;
FIG. 2 is a diagram showing an example of the structure of a nucleic acid amplification analyzer according to one embodiment;
FIG. 3 is a diagram showing an example of the structure of a pretreatment unit according to one embodiment;
FIG. 4 is a diagram showing an example of the structure of a measurement unit according to one embodiment;
FIG. 5 is a diagram showing an example of the structure of a data processing unit according to one embodiment;
FIG. 6 is a flowchart showing a series of processes by the CPU of the data processing unit according to one embodiment;
FIG. 7 is a diagram showing an example of an amplification reaction curve by a nucleic acid amplification reaction in the nucleic acid amplification analyzer according to one embodiment;
FIG. 8 is a flow chart showing a first example of the success/failure determination process of nucleic acid amplification according to one embodiment;
FIG. 9 is a flowchart showing a second example of the success/failure determination process of nucleic acid amplification according to one embodiment;
FIG. 10 is a diagram showing an example of a transmitted light curve corresponding to a nucleic acid amplification reaction in which calibrators having different nucleic acid concentrations are measured according to one embodiment;
FIG. 11 is a diagram showing an example of a transmitted light curve corresponding to a nucleic acid amplification reaction in which different measurement samples are measured according to one embodiment;
FIG. 12 is a diagram showing an example of a transmitted light curve corresponding to a nucleic acid amplification reaction with a specific calibrator as a measurement target according to one embodiment; and
FIG. 13 is a diagram showing an example of a measurement value display screen displaying an example of a measurement value corresponding to the amount of nucleic acid amplified by the nucleic acid amplification reaction according to one embodiment.

### DESCRIPTION OF THE EMBODIMENTS OF THE INVENTION

Hereinafter, preferred embodiments of the present invention will be described with reference to the drawings. Note that identical elements are designated by the same reference numeral, and duplicate description will be omitted. Unless otherwise specified, the positional relationship such as up, down, left, and right shall be based on the positional relationship shown in the drawings. The dimensional ratios in the drawings are not limited to the ratios shown. In addition, the following embodiments are examples for explaining the present invention, and the present invention is not limited to these embodiment.

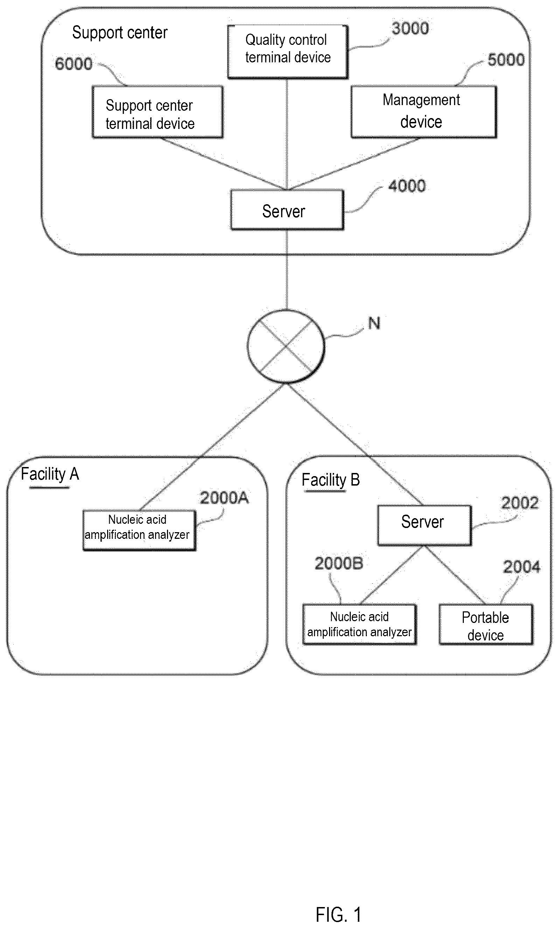

FIG. 1 is a diagram showing the structure of a nucleic acid amplification analysis system according to an embodiment. As shown in FIG. 1, the nucleic acid amplification analysis system 1 includes a quality control terminal device 3000, a server 4000, a management device 5000, a support center terminal device 6000, a nucleic acid amplification analyzer 2000A installed in a facility A, a nucleic acid amplification analyzer 2000B installed in a facility B, a server 2002, and a terminal device 2004. Each of the facilities A and B includes, for example, a clinical laboratory, a clinical laboratory center, or the like. When facilities A and B are not distinguished, they are referred to as "facility" below. The number of facilities according to one embodiment may be two, or less or three or more. When the nucleic acid amplification analyzers 2000A and 2000B are not discriminated, they are hereinafter referred to as "nucleic acid amplification analyzer 2000" (nucleic acid amplification success/failure determination device). The number of nucleic acid amplification analyzers 2000 arranged in the facility may be two or more.

The nucleic acid amplification analyzer 2000 is communicably connected to the quality control terminal device 3000, the management device 5000, and the support center terminal device 6000 via a predetermined communication network N (network). This connection is via server 4000 at the support center.

The quality control terminal device 3000 in the support center is a device for managing the analysis accuracy (including the success/failure determination accuracy of nucleic acid amplification) of the nucleic acid amplification analyzer 2000. The specific configuration of the quality control terminal device 3000 will be described later. The management device 5000 is a terminal operated by an administrator who manages the support center, and executes various processes related to the management of the entire support center. The support center terminal device 6000 is, for example, a device operated by a person working at the support center.

The nucleic acid amplification analyzer 2000B in facility B makes a communication connection with another device via the server 2002 in the facility B. Note that the nucleic acid amplification analyzer 2000B also may make a communication connection with another device without going through the server 2002. The mobile terminal 2004 displays various information output by the nucleic acid amplification analyzer 2000B. The mobile terminal 2004 includes, for example, a tablet terminal device.

The nucleic acid amplification analyzer 2000 acquires a measurement value (for example, light amount, absorbance, turbidity, and the like) corresponding to the amount of nucleic acid amplified by the nucleic acid amplification reaction of the measurement sample containing nucleic acid. The nucleic acid amplification analyzer 2000 acquires a determination result for determining the success or failure of the nucleic acid amplification reaction based on the acquired measurement value. In addition to or instead of the success/failure determination process of the nucleic acid amplification reaction, the nucleic acid amplification analyzer 2000 also may execute the generation of quality control data carried out by the quality control terminal device 3000, which will be described later.

The nucleic acid amplification analyzer 2000 also may acquire calibration data obtained by measuring a standard sample (for example, a calibrator) in order to monitor the analysis accuracy of the nucleic acid amplification analyzer 2000 to be managed. The calibration data may include, for example, the measurement data of the calibrator, the rise time of the turbidity thereof, and the calibration curve created from the measurement data. The nucleic acid amplification analyzer 2000 also may acquire operational data and the like of the nucleic acid amplification analyzer 2000. The operational data are, for example, data obtained by monitoring the operation of the nucleic acid amplification analyzer 2000 during sample measurement. The operational data includes, for example, contains at least one type of data to be selected from a group consisting of capacitance, quantitative count (volume of liquid), reagent suction and discharge pressures, ambient temperature, remaining reagent amount, error code, error content, error occurrence date and time, and status.

The nucleic acid amplification analyzer 2000 transmits at least one of the measurement value, the determination result, the calibration data, or the operational data to the quality control terminal device 3000.

The quality control terminal device 3000 generates quality control data. The quality control terminal device 3000 acquires the determination result of determining the success or failure of the nucleic acid amplification reaction from each of the plurality of nucleic acid amplification analyzers 2000. The quality control terminal device 3000 calculates the analysis accuracy from the plurality of determination results acquired from the plurality of nucleic acid amplification analyzers 2000, and generates quality control data. Hence, the quality control terminal device 3000 evaluates whether the measurement value in the nucleic acid amplification reaction of the measurement sample containing nucleic acid can be accurately obtained, or whether the success or failure of the nucleic acid amplification reaction can be accurately determined.

Note that in addition to or instead of the above-mentioned quality control data generation process, the quality control terminal device 3000 (determination device) may execute a success/failure determination process of the nucleic acid amplification reaction carried out by the nucleic acid amplification analyzer 2000. In this case, the quality control terminal device 3000 acquires, for example, a measurement value corresponding to the amount of amplified nucleic acid in a state in which the nucleic acid amplification reaction has reached a saturation state, based on the output from the nucleic acid amplification analyzer 2000, and determines the success or failure of the nucleic acid amplification reaction based on the acquired measurement value.

In one embodiment, the sample includes a human specimen. A sample of a mammal also may be used as a sample. Mammals include, for example, monkeys, dogs, cats, rabbits and the like. The sample is not limited insofar as the specimen can be collected from the subject. Samples include, for example, tissues, cells, serum, plasma, urine, cerebrospinal fluid, ascites, pleural effusion, saliva, gastric juice, pancreatic juice, bile, milk, interstitial fluid and the like.

The test item may be, for example, a test for detecting a lesion. The lesion is, for example, a malignant tumor. Malignant tumors include, for example, respiratory malignancies originating from the trachea, bronchi, lungs and the like (including lung cancers such as squamous cell lung cancer, small cell lung cancer, large cell lung cancer, adenocarcinoma); gastrointestinal malignant tumors originating from the nasopharynx, esophagus, stomach, duodenum, jejunum, ileum, cecum, appendix, ascending colon, transverse colon, sigmoid colon, rectum or anal region; liver cancer; pancreatic cancer; urological malignancies originating from the bladder, ureter or kidney; female reproductive system malignancies originating from the ovary, fallopian tubes, uterus; breast cancer; prostate cancer; skin cancer; endocrine malignancies such as hypothalamus, pituitary gland, thyroid gland, parathyroid gland, adrenal gland; central nervous system malignancies; including solid tumors such as malignant tumors originating from bone and soft tissues. Malignant tumors include, for example, respiratory epithelial malignancies such as lung cancer (squamous cell carcinoma, small cell carcinoma, large cell carcinoma, adenocarcinoma); gastrointestinal epithelial malignancies such as gastric cancer, duodenal cancer, colon cancer (sigmoid colon cancer, rectal cancer and the like); liver cancer; pancreatic cancer; bladder cancer; thyroid cancer; ovarian cancer; breast cancer; prostate cancer.

As a measurement principle, a nucleic acid detection method for measuring the presence or absence of a specific DNA sequence and the expression level of mRNA can be mentioned. In the nucleic acid detection method, for example, the expression level of a cancer gene or the like can be measured by the RT-LAMP (Reverse Transcription-Loop-Mediated Isothermal Amplification) method, the quantitative RT-PCR method, the microarray method, the RNA-Seq method or the like . In the nucleic acid detection method, abnormality (mutation or the like) such as EGFR (epidermal growth factor receptor) gene or the like can be detected by the PCR method, the sequencing method or the like.

The nucleic acid amplification analyzer 2000 according to the present embodiment is a gene amplification detection device that measures the presence or absence of a specific DNA sequence and the expression level of mRNA by a nucleic acid detection method.

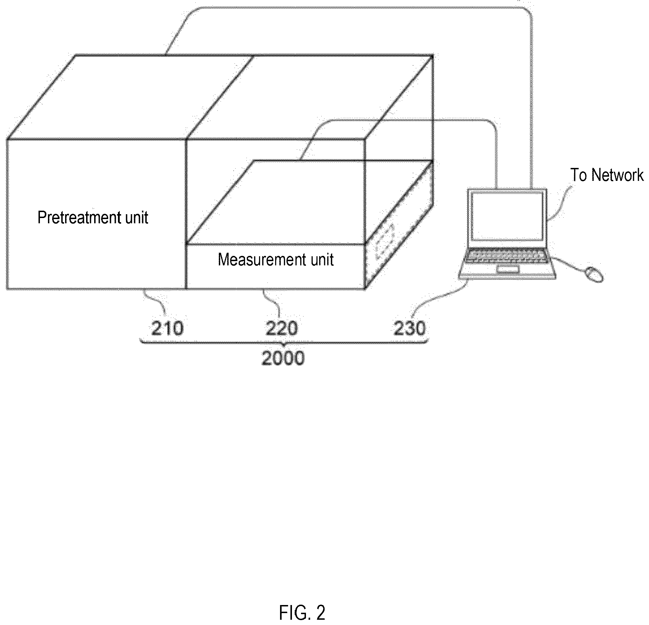

FIG. 2 is a diagram showing an example of the structure of the nucleic acid amplification analyzer according to the present embodiment. As shown in FIG. 2, the nucleic acid amplification analyzer 2000 can obtain a measurement value corresponding to the presence or amount of a target nucleic acid (nucleic acid) contained in this sample by using a tissue or the like extracted from the subject as a sample. More specifically, the nucleic acid amplification analyzer 2000 is used, for example, as a genetic diagnosis system for cancer lymph node metastasis. The nucleic acid amplification analyzer 2000 performs pretreatment (for example, homogenization, extraction treatment and the like) on lymph nodes (samples) excised from the human body to prepare a solubilized extract as a measurement sample for nucleic acid detection.

The nucleic acid amplification analyzer 2000 amplifies the target nucleic acid present in the measurement sample by the LAMP method. The nucleic acid amplification analyzer 2000 detects or quantifies the target nucleic acid (oncogene; mRNA) by measuring the turbidity of the solution generated by the amplification. The nucleic acid amplification analyzer 2000 measures the amount of light or the absorbance of light that passes through a turbid solution due to amplification. In this way, the nucleic acid amplification analyzer 2000 acquires measurement values (for example, light amount, absorbance, turbidity, etc.) corresponding to the amount of nucleic acid amplified by the nucleic acid amplification reaction of the measurement sample containing nucleic acid.

The nucleic acid amplification analyzer 2000 includes, for example, a pretreatment unit 210 for preparing a measurement sample by performing pretreatment such as homogenization on a sample obtained from a human body or the like, and a measurement unit 220 (nucleic acid amplification unit and detection unit) that detects the target nucleic acid contained in the measurement sample. The nucleic acid amplification analyzer 2000 also includes a data processing unit 230 for performing data processing, data communication, and the like. The data processing unit 230 (control unit) serves as a control device that receives measurement data from each of the pretreatment unit 210 and the measurement unit 220, and transmits operation instruction signals or the like to the pretreatment unit 210 and the measurement unit 220. That is, the pretreatment unit 210 and the data processing unit 230 function as pretreatment devices, and the measurement unit 220 and the data processing unit 230 function as nucleic acid detection devices. The data processing unit 230 is connected to the communication network N shown in FIG. 1, and executes data transmission/reception between the above-mentioned quality control terminal device 3000 or the server 4000.

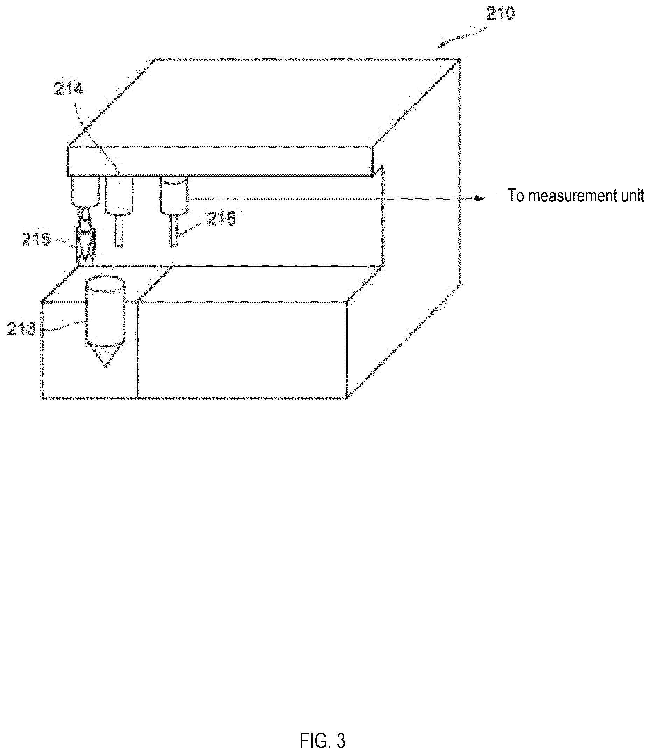

FIG. 3 is a diagram showing an example of the structure of the pretreatment unit according to the present embodiment. As shown in FIG. 3, the pretreatment unit 210 includes a sample setting unit 213 for setting a container containing a sample and a reagent addition unit 214 for adding a pretreatment reagent to the sample container set in the sample setting unit 213 a blender 215 for homogenizing the sample, and a pipette 216 for dispensing the homogenized (pretreated) measurement sample.

When the pretreatment unit 210 receives the measurement start instruction signal from the data processing unit 230, the pretreatment reagent is added to the sample of the sample setting unit 213 (pretreatment reagent addition treatment), and the sample is homogenized by the blender 215 to prepare a measurement sample. Then, the measurement sample is suctioned by the pipette 216, the pipette 216 moves to the measurement unit 220, and the sample is injected into the sample container 22 set in the measurement unit 220.

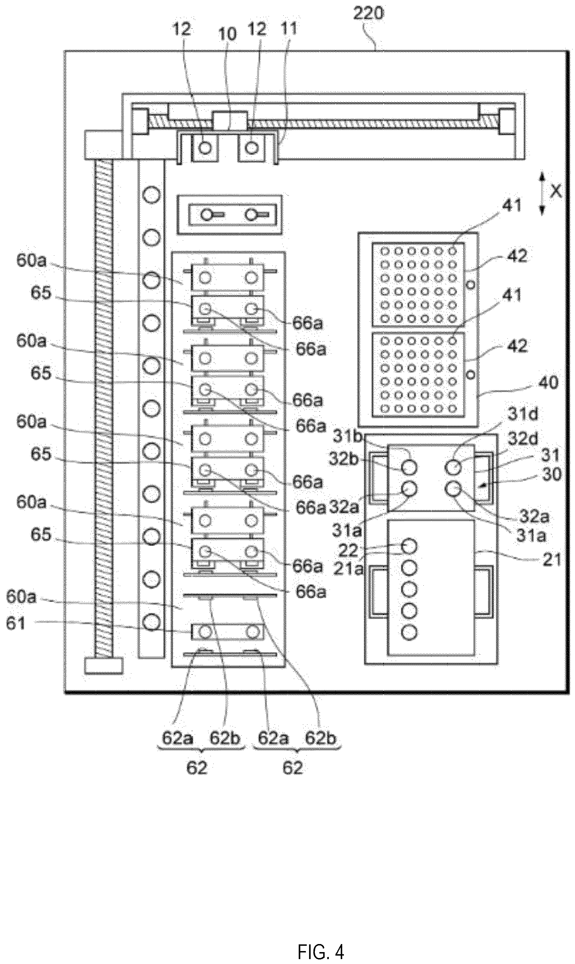

FIG. 4 is a diagram showing an example of the structure of the measurement unit 220 according to the present embodiment. Specifically, FIG. 4 is a plan view showing an example of the structure of the measurement unit 220. Details of the measuring unit 220 are described in U.S. Patent Publication No. 2005-0042138 and are incorporated herein by reference. Here, the structure and operation of the measurement unit 220 will be briefly described. First, the pipette that has moved from the pretreatment unit 210 injects the pretreated sample into the sample container 22 set in the sample container set hole 21a of the sample container base 21.

A primer reagent container 32a containing a primer reagent (nucleic acid amplification reagent) of CK19 (cytokeratin 19), which is a target nucleic acid, and an enzyme reagent container 32b containing an enzyme reagent are set in the primer reagent container set hole 31a and the enzyme reagent container set hole 31b on the left side of the front of the reagent container setting unit 30. The primer reagent container set hole 31a on the front right side of the reagent container setting unit 30 accommodates a primer reagent container 32a containing a primer reagent (nucleic acid amplification reagent) of Arabidopsis (hereinafter referred to as "Arabide"). Further, an arabide solution container 32d containing a predetermined amount of arabide is set in the arabide container set hole 31d on the right side of the front surface.

Two racks 42, each containing 36 disposable pipette tips 41, are fitted in the tip setting unit 40. Two cell units 66a of the detection cell 65 are set in the two detection cell set holes of the reaction unit 61 of each reaction detection block 60a.

In this state, when the operation of the measurement unit 220 starts and after the arm 11 of the dispensing mechanism 10 is moved from the initial position to the tip setting unit 40, two syringe units 12 of the dispensing mechanism 10 are moved downward at the tip setting unit 40. In this way the tip ends of the nozzles of the two syringe units 12 are press-fitted into the upper opening of the two pipette tips 41, so that the pipette tips 41 are automatically attached to the tip ends of the nozzles of the two syringe units 12. Then, after the two syringe units 12 are moved upward, the arm 11 of the dispensing mechanism 10 is moved in the X axis direction above the two primer reagent containers 32a containing arabide primer reagent and a target nucleic acid set in the reagent container setting base 31. When the two syringe units 12 are moved in the downward direction, the tips of the two pipette tips 41 attached to the nozzles of the two syringe units 12 are respectively inserted in the liquid surface of the arabide and target nucleic acid primers in the two primer reagent containers 32a. Then, the primer reagents of CK19 and arabido in the two primer reagent containers 32a are suctioned by the pump section of the syringe unit 12.

After the suction of the primer reagents, and after the two syringe units 12 are moved upward, the arm 11 of the dispensing mechanism 10 moves above the reaction detection block 60a positioned on the innermost side (the front side of the apparatus). [In this case, the arm 11 of the dispensing mechanism 10 is moved so as not to pass above the other second to fifth reaction detection blocks 60a from the inner side. Then, in the reaction detection block 60a on the innermost side, the two syringe units 12 are moved in the downward direction so that the two pipette tips 41 attached to the nozzles 12a of the two syringe units 12 are inserted into the two cell units 66a of the cell 65. Then, using the pumps of the syringe unit 12, the two primer reagents CK19 and arabide are respectively discharged into two cell units 66a (primer reagent dispensing process).

Thereafter, the pipette tips 41 are discarded, and two new pipette tips 41 are automatically attached to the tips of the nozzles of the two syringe units 12, and in substantially the same operation as above, the enzyme reagent is discharged into the two cell units 66a of the detection cell 65 (enzyme reagent dispensing process). Thereafter, in a similar manner, the arabide solution in the arabide solution container 32d is discharged to the two cell units 66a of the detection cell 65. Then, similarly, the sample (measurement sample) of the sample container 22 is discharged to the two cell units 66a of the detection cell 65 (sample dispensing process). In this way the sample for detecting the target nucleic acid is adjusted in one cell unit 66a of the detection cell 65, and the sample for detecting arabido is adjusted in the other cell unit 66a.

The lid closing operation of the detection cell 65 is performed after the primer reagent, the enzyme reagent, the arabide solution and the sample are discharged into the cell unit. After the lid closing operation is completed, the liquid temperature in the detection cell 65 is heated from about 20°C to about 65°C by using the Peltier module of the reaction unit 61, whereby the target gene (CK 19) and arabide are amplified by the LAMP method. Then, white turbidity due to magnesium pyrophosphate produced along with amplification is detected by a turbidimetric method. Specifically, light having a diameter of about 1 mm is emitted from the LED light source unit 62a of the turbidity detection unit 62 on the cell unit 66a of the detection cell 65 during the amplification reaction via the light irradiation groove of the reaction unit 61. Then, the irradiated light is received by the photodiode light receiving unit 62b. In this way the turbidity in the cell unit 66a of the detection cell 65 during the amplification reaction and the amount of light received by the photodiode light receiving unit 62b are detected (monitored) in real time as measurement values. The measurement values regarding CK19 and arabide measured by the photodiode light receiving unit 62b are transmitted from the measurement unit 220 to the data processing unit 230.

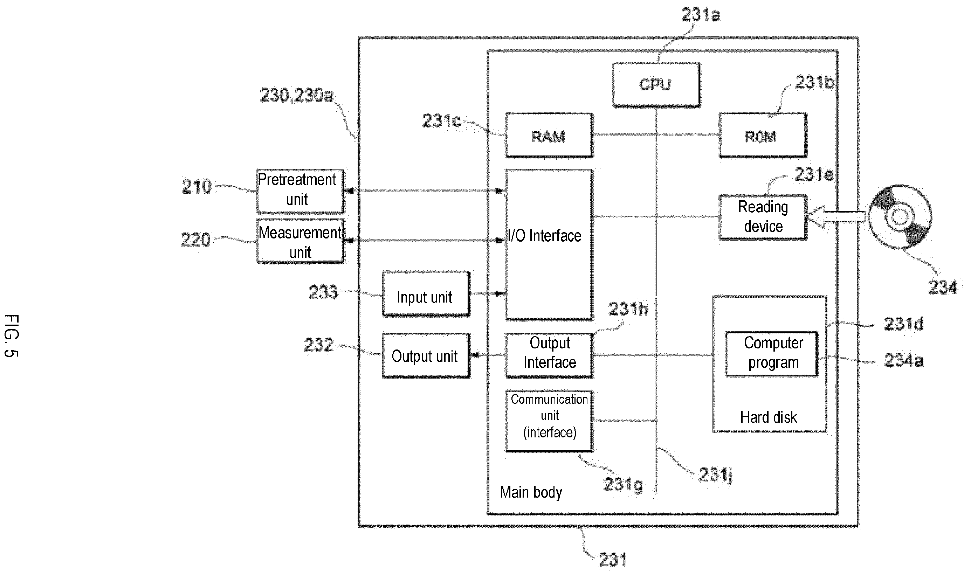

FIG. 5 is a diagram showing an example of the structure of a data processing unit according to one embodiment; The data processing unit 230 is realized by the computer 230a. As shown in FIG. 5, the computer 230a includes a main body 231, an output unit 232, and an input unit 233. The main body 231 is, for example, a processing unit 231a (CPU: Central Processing Unit, GPU: Graphics Processing Unit, or MPU: Micro Processing Unit), a ROM (read-only memory) 231b, a main storage unit (RAM: Random memory) 231b, an auxiliary storage unit (hard disk) 231d, a reading device 231e, an input/output interface 231f, a communication interface 231g, and an output interface 231h. In the main body 231 the processing unit 231a, ROM 231b, main storage unit 231c, auxiliary storage unit 231d, reading device 231e, input/output interface 231f, communication interface 231g, and output interface 231h are connected by a bus 231j. The main storage unit 231c and the auxiliary storage unit 231d are collectively referred to as a storage unit.

The reading device 231e can read a computer program 234a for causing the computer to function as the information processing unit 230 from a portable recording medium 234, and install the computer program 234a in the hard disk 231d.

The preprocessing unit 210, and the measuring unit 220 are respectively connected via cables to the input/output interface 231f. The input/output interface 231f is connected to the pretreatment unit 210 and the measurement unit 220 so so as to allow communication of data and the output of control signals to the preprocessing unit 210 and the measurement unit 220. Upon receiving such a control signal, the pretreatment unit 210 and the measurement unit 220 decode the control signal and drive the actuators of each mechanical unit in response to the control signal. Measurement data can be transmitted from the pretreatment unit 210 and the measurement unit 220 to the data processing unit 230, and the CPU 231a performs predetermined processing when the data processing unit 230 receives the measurement data.

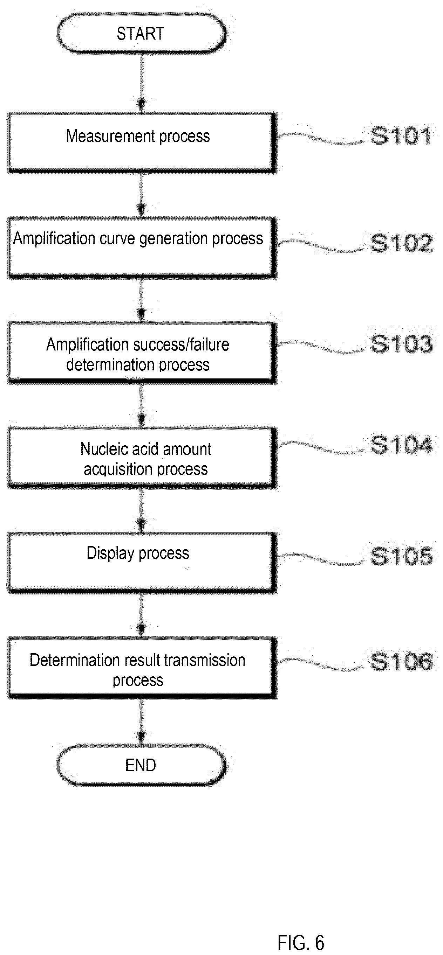

FIG. 6 is a flowchart showing a series of processes performed by the CPU 231a. The CPU 231a controls each part of the measurement unit 220 and executes the measurement process (step S101). The flow of the measurement process is as described with reference to FIG. 4. When the measurement process in step S101 is completed, the CPU 231a executes the amplification curve generation process (step S102).

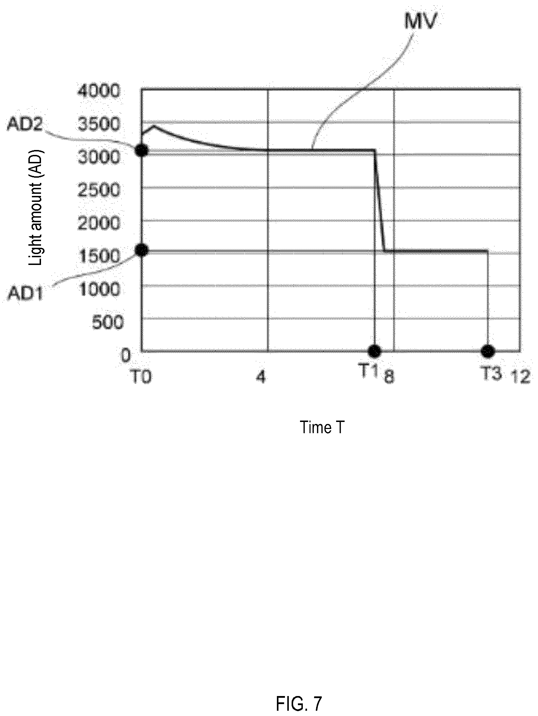

FIG. 7 is a diagram showing an example of the amplification curve MV generated by the amplification curve generation process. The CPU 231a generates an amplification curve MV having the amount of light received by the photodiode light receiving unit 62b as the vertical axis and the elapsed time from the start of light detection by the photodiode light receiving unit 62b as the horizontal axis. The CPU 231a generates an amplification curve MV for each of the twelve turbidity detection units 62.

Next, the CPU 231a executes a nucleic acid amplification success/failure determination process (step S103).

Nucleic Acid Amplification Success/Failure Determination Process (step S103)

### Example 1

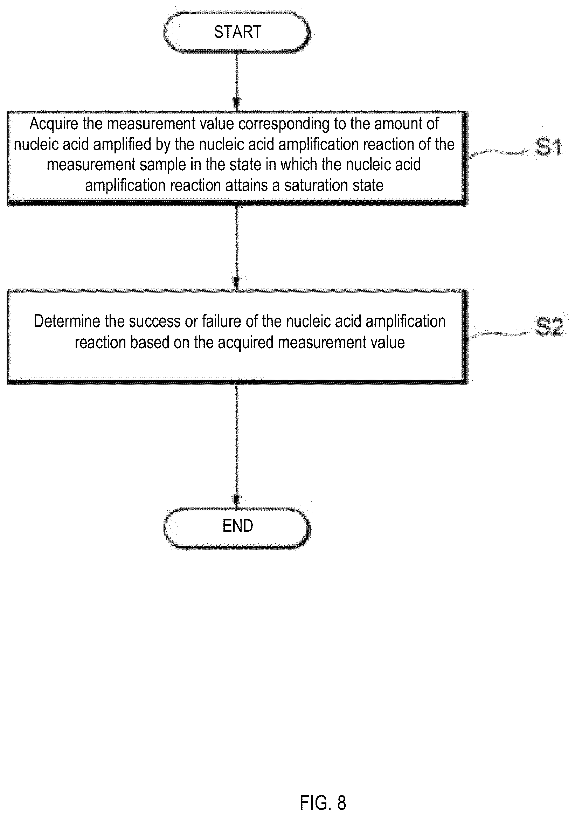

FIG. 8 is a flowchart showing a first example of the nucleic acid amplification success/failure determination process (step S103). As shown in FIG. 8, the CPU 231a acquires a measurement value corresponding to the amount of nucleic acid amplified by the nucleic acid amplification reaction of the measurement sample containing nucleic acid in a state in which the nucleic acid amplification reaction has reached a saturation state (step S1). Specifically, in the amplification curve MV shown in FIG. 7, the light amount AD1 is acquired at the time T3 corresponding to the state in which the nucleic acid amplification reaction has reached the saturation state. The CPU 231a determines the success or failure of the nucleic acid amplification reaction based on the acquired measurement value (step S2). Specifically, the success or failure of the nucleic acid amplification reaction is determined by comparing the light amount AD1 acquired in step S1 with the past light amount of the measurement sample in which the nucleic acid amplification reaction was successful (the light amount obtained in the state in which the nucleic acid amplification reaction has reached the saturation state). This determination will be described later.

### Example 2

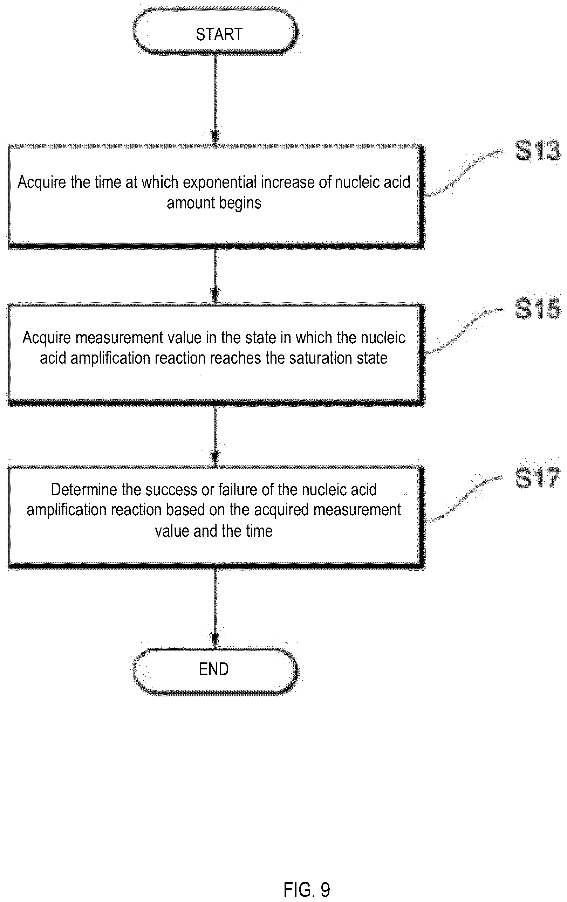

FIG. 9 is a flowchart showing a second example of the nucleic acid amplification success/failure determination process (step S103). As shown in FIG. 9, the CPU 231a acquires the time (amplification rise time) at which the exponential increase in the amount of nucleic acid starts (step S13). Specifically, in the amplification curve MV shown in FIG. 7, the time T1 at which the light amount AD suddenly decreases is acquired. Next, the CPU 231a acquires a measurement value in a state in which the nucleic acid amplification reaction has reached a saturation state (step S15). Specifically, in the amplification curve MV shown in FIG. 7, the light amount AD1 is acquired at the time T3 corresponding to the state in which the nucleic acid amplification reaction has reached the saturation state. The CPU 231a determines the success or failure of the nucleic acid amplification reaction based on the acquired measurement value (AD1) and the time T1 (step S17). This determination will be described later.

### Nucleic Acid Amplification Success/Failure Determination Process (step S12, S17)

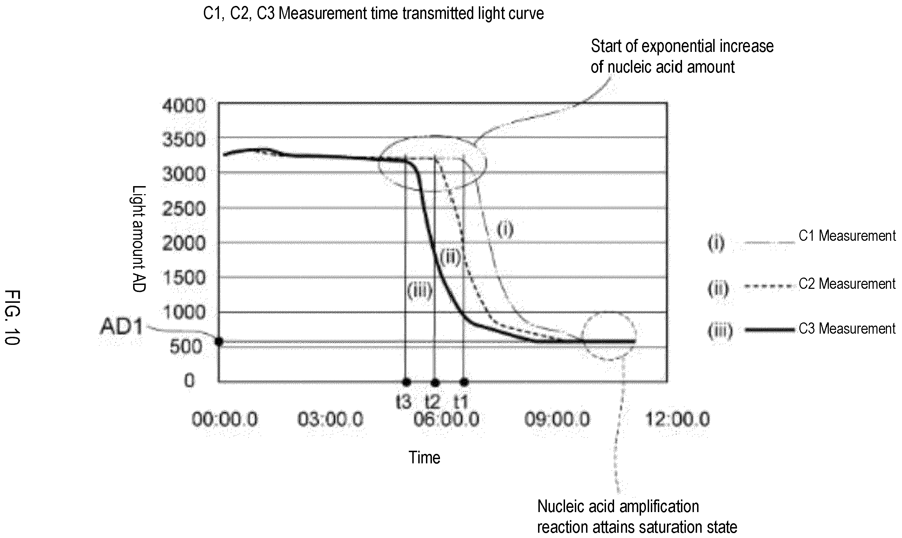

FIG. 10 is a diagram showing a transmitted light curve corresponding to a nucleic acid amplification reaction in which calibrators (C1, C2, and C3) having different nucleic acid concentrations are measured by the nucleic acid amplification analyzer 2000 according to the present embodiment. As shown in FIG. 10, the amplification rise time in the measurement results of the calibrators C1, C2, and C3 (amplification curves (hereinafter referred to as transmitted light curves) (i), (ii) and (iii)), depends on the nucleic acid concentration. That is, the amplification rise time for the measurement sample including the calibrator C1 is time t1, the amplification rise time for the measurement sample including the calibrator C2 is time t2, and the amplification rise time for the measurement sample including the calibrator C3 is time t3. On the other hand, in the measurement results (transmitted light curves (i), (ii) and (iii)) of the calibrators C1, C2, and C3, the amount of light (turbidity) AD1 in the state in which the nucleic acid amplification reaction has reached the saturation state is the same. Note that in the example shown in FIG. 10 the nucleic acid amplification reaction was successful for each of the plurality of measurement samples.

The "state in which the nucleic acid amplification reaction has reached a saturation state" is a state in which the increase in the amount of nucleic acid has substantially disappeared, or a state in which the increase in the amount of nucleic acid has reached a period estimated to be substantially eliminated.

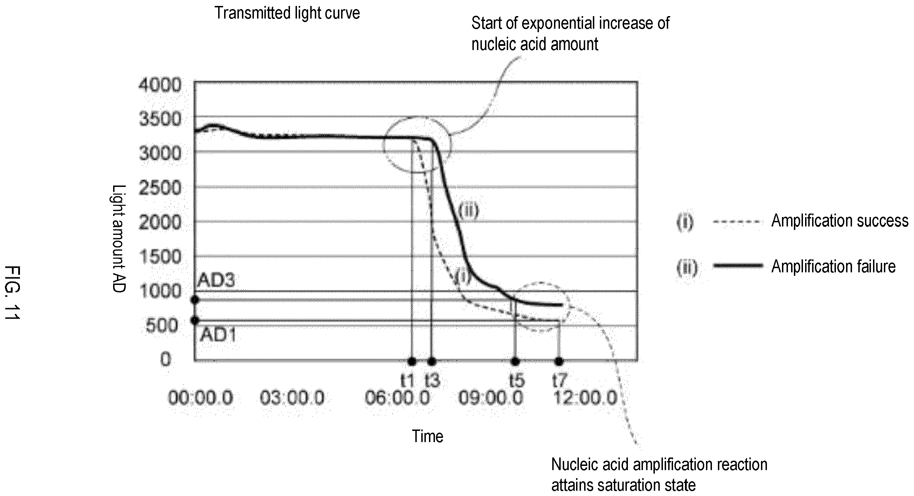

FIG. 11 is a diagram showing a transmitted light curve corresponding to a nucleic acid amplification reaction in which two measurement samples are measured by the nucleic acid amplification analyzer 2000 according to the present embodiment. The examples shown in FIG. 11 include the past measurement results (transmitted light curve (i)) of the measurement sample in which the nucleic acid amplification reaction was successful and the measurement results (transmitted light curve (ii)) of the current measurement sample in which the nucleic acid amplification reaction failed.

The success or failure of nucleic acid amplification is determined by comparing the measurement value (AD3 in the example of FIG. 8) acquired when the nucleic acid amplification reaction has reached the saturation state (between time t5 and time t7), and other measurement values (AD1 in the example of FIG. 11) obtained in the state in which the nucleic acid amplification reaction successfully reached the saturation state for the measurement sample in the past. If the current measurement value is in agreement with the past measurement value in the successful nucleic acid amplification reaction, it is determined that the current nucleic acid amplification reaction was successful. Even if the current measurement value does not match the past measurement value in the successful nucleic acid amplification reaction, the current nucleic acid amplification reaction may be determined to be successful when the difference between the current measurement value and the past measurement value is within a predetermined value, or the current measurement value or when the ratio of the current measurement value to the past measurement value is within a predetermined range. The example of FIG. 11 shows that the difference between the current measurement value AD3 and the past measurement value AD1 exceeds a predetermined value, indicating nucleic acid amplification has failed in this measurement. According to this structure, the accuracy of the nucleic acid amplification success/failure determination of the nucleic acid amplification reaction is improved by comparing the acquired measurement value AD3 with the measurement value AD1 obtained in the state in which the successful nucleic acid amplification reaction has reached the saturation state of another measurement sample in the past.

The measurement sample in which nucleic acid amplification was successful in the past is preferably the measurement sample measured immediately before the current measurement sample. In this way the possibility that the current measurement sample and other measurement samples are measured in the same measurement environment is increased, so that the accuracy of the nucleic acid amplification reaction success/failure determination is further improved.

The success or failure of nucleic acid amplification also can be determined by comparing the measurement value (AD1 or AD3) acquired in the state in which the nucleic acid amplification reaction has reached the saturation state (between time t5 and time t7) with a predetermined threshold value.

Note that the acquisition of the measurement value is executed at a predetermined timing (time t7 in the example of FIG. 11) in the state in which the nucleic acid amplification reaction has reached the saturation state. The acquisition of the measurement value also may be executed a plurality of times in a state where the nucleic acid amplification reaction has reached a saturation state.

In the nucleic acid amplification success/failure determination, in addition to the determination process based on the measurement value acquired in the state in which the nucleic acid amplification reaction has reached the saturation state, a determination process based on information related to the amplification rise time also may be included.

The nucleic acid amplification analyzer 2000 acquires the amplification rise time (time when a substantial increase in the amount of nucleic acid starts; in the example of FIG. 11, times t1 and t3) in determining the success or failure of nucleic acid amplification, and determines the success or failure of the nucleic acid amplification reaction based on the measurement value AD3 and the amplification rise time t3. Specifically, in the nucleic acid amplification success/failure determination, the measurement values this time (for example, the measurement value AD3 and the amplification rise time t3) are compared with the past measurement values of the successful nucleic acid amplification reaction. Here, the past measurement values of the successful nucleic acid amplification reaction are, for example, the measurement value AD1 acquired in the state wherein which the nucleic acid amplification reaction has reached the saturation state for other measurement samples in the past, and the amplification rise time t1 in other measurement samples in the past. In the determination, if both the difference between the measurement value AD3 and the measurement value AD1 and the difference between the time t3 and the time t1 are within a predetermined range, it is determined that the nucleic acid amplification is successful (other combinations result in failure); alternatively, when both the difference between the measurement value AD3 and the measurement value AD1 and the difference between the time t3 and the time t1 exceed a predetermined range, it is determined that the nucleic acid amplification fails (other combinations are successful). According to this configuration, in the nucleic acid amplification success/failure determination, information related to the amplification rise time is also taken into consideration in addition to the measurement values acquired when the nucleic acid amplification reaction has reached a saturation state, so as to improve the accuracy of the nucleic acid amplification reaction success/failure determination. Note that when the PCR method is used as the nucleic acid amplification method, the number of amplifications can be used instead of the time t1 and the time t3.

In the nucleic acid amplification analyzer 2000, determining the success or failure of nucleic acid amplification includes evaluating the state of the nucleic acid amplification reagent for amplifying nucleic acid.

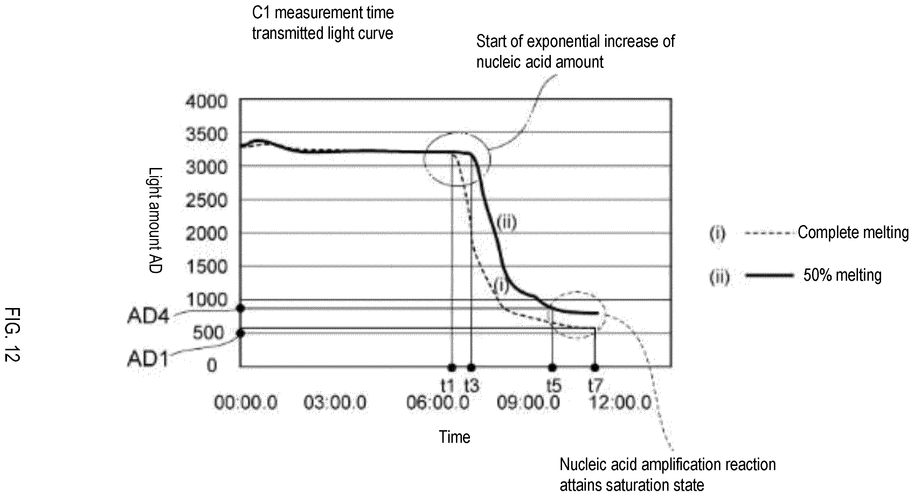

FIG. 12 is a diagram showing a transmitted light curve corresponding to a nucleic acid amplification reaction with a specific calibrator as a measurement target by the nucleic acid amplification analyzer 2000 according to the present embodiment. For example, in the LAMP method, a cryopreserved nucleic acid amplification reagent is thawed and stirred for use. A primer-containing reagent completely melted and stirred and a primer-containing reagent 50% melted were prepared, and a specific calibrator C1 was used as a measurement target. As a result, as shown in FIG. 12, in a transmitted light curve (i) corresponding to the primer-containing reagent completely melted and stirred and a transmitted light curve (ii) corresponding to the primer-containing reagent about 50% melted, a different occurred in the amplification rise times (time t1 and time t4), and a clear difference occurred in the turbidity (light intensity) (measurement values AD1 and AD4) at the time when the nucleic acid amplification reaction is saturated, that is, the amount of amplification product. This is because at least one of the melted state and the stirred state of the primer-containing reagent is insufficient, such that the primer is insufficient in the amplification reaction field, and the reaction components are depleted. In this way the nucleic acid amplification analyzer 2000 can evaluate the state (at least one of the melting state and the stirring state) of the nucleic acid amplification reagent in the nucleic acid amplification reaction based on the amount of light at the time when the nucleic acid amplification reaction is saturated.

In the nucleic acid amplification success/failure determination, it is preferable that the current measurement sample to be determined and the other measurement samples are nucleic acids amplified using a nucleic acid amplification reagent of the same production lot. According to this structure, in the method for determining the success or failure of nucleic acid amplification, the success or failure of the nucleic acid amplification reaction is determined by comparing the measurement values in different measurement samples in which nucleic acids are amplified using nucleic acid amplification reagents of the same production lot. In this way the determination is not affected by the difference in reagents (for example, the difference in the mixing ratio of the primers, the difference in the performance of the primers, and the like) due to the difference in the production lot, and the accuracy of the nucleic acid amplification reaction determination is further improved.

When the nucleic acid amplification analyzer 2000 further includes a plurality of detection units that detect measurement values corresponding to the amount of nucleic acid, the success or failure of nucleic acid amplification may be determined by comparing the acquired measurement values with the measurement values acquired in the state in which the nucleic acid amplification reaction has reached the saturation state for other measurement samples in the past. According to this structure, the nucleic acid amplification analyzer 2000 determines the success or failure of the nucleic acid amplification reaction by comparing the acquired measurement value with the measurement value acquired in the state in which the nucleic acid amplification reaction has reached the saturation state for other measurement samples in the past for each of the respective plurality of detection units. Therefore, in determining the success or failure of the nucleic acid amplification reaction, for example, the accuracy of nucleic acid amplification reaction determination is further improved since the determination is not affected by performance variations among a plurality of detection units (reaction detection block 60a) because the current measurement value is compared with the measurement value acquired in the past with the same detection unit (reaction detection block 60a).

### Nucleic Acid Amount Acquisition Process (step S104)

Returning to FIG. 6, the CPU 231a executes the nucleic acid amount acquisition process (step S104). Specifically, the CPU 231a acquires the light amount AD at the time T1 (amplification rise time) of the amplification curve in FIG. 7, and the acquired light amount AD is applied to a calibration curve prepared by using a plurality of calibrators having known nucleic acid amounts and different nucleic acid amounts to obtain the nucleic acid amount.

### Display Process (step S105)

The CPU 231a outputs information regarding the success or failure of the nucleic acid amplification reaction by the output unit 232. The information regarding the success or failure of the nucleic acid amplification reaction is information including whether the nucleic acid amplification reaction for a specific measurement sample succeeded or failed. Since the information related to the success or failure of the acid amplification reaction is output, the measurer can easily confirm the information on the success or failure of the nucleic acid amplification reaction. Note that the information regarding the success or failure of the nucleic acid amplification reaction may be text information, image information, or voice information.

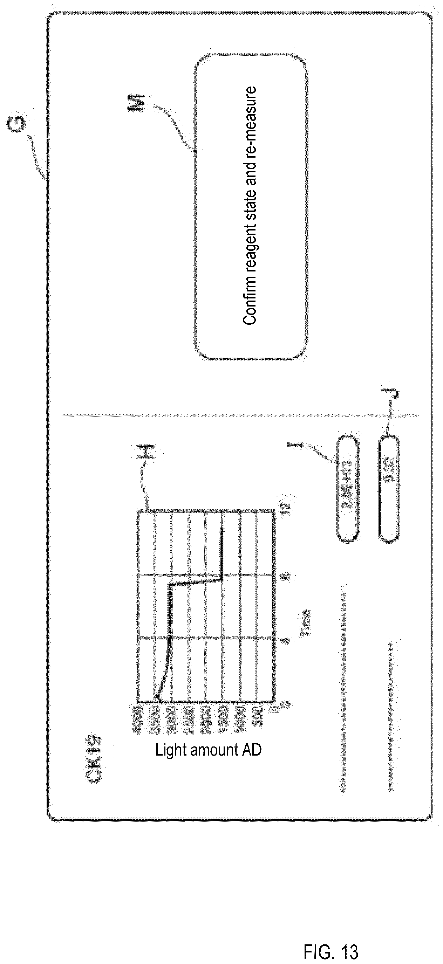

FIG. 13 is a diagram showing an example of the measurement value display screen G displayed by the output unit 232 of the nucleic acid amplification analyzer 2000 according to the present embodiment. As shown in FIG. 13, the measurement value display screen G includes an amplification curve display area H for displaying the amplification curve MV, the nucleic acid amount display area I for displaying the nucleic acid amount acquired in step S104, and the amplification rise time display area J for displaying the amplification rise time, and a message display area M for displaying a message prompting to confirm the state of the nucleic acid amplification reagent for amplifying nucleic acid. In the message display area M, when it is determined that the nucleic acid amplification reaction has failed as a result of the determination in step S2 or step S17, a message prompting to confirm the state of the nucleic acid amplification reagent for amplifying the nucleic acid is displayed. The message displayed in the message display area M may be, for example, "Check the state of the reagent and measure again." According to this structure, the measurer can confirm the state of the nucleic acid amplification reagent at an appropriate timing. Note that, as described above, the message M may be displayed on the measurement value display screen G, or may be displayed on a screen different from the measurement value display screen G. In addition to or instead of the message, a mark indicating a warning may be displayed, or an warning or voice notification may be given.

### Determination Result Transmission Process (step S106)

Returning to FIG. 6, the CPU 231a executes the nucleic acid amount acquisition process (step S106). Specifically, the CPU 231a transmits the amplification curve MV generated in step S102, the nucleic acid amplification success/failure determination result acquired in step S103, and the amount of nucleic acid acquired in step S104 to the quality control terminal device 3000 of the support center shown in FIG. 1. The transmission timing is preferably immediately after the processing execution in step S104, but may be within a certain period of time (for example, within 5 minutes). In this way the support staff operator of the quality control terminal device 3000 can grasp the operating status of the nucleic acid amplification analyzer 2000. When the nucleic acid amplification fails, the support staff can quickly support the user of the nucleic acid analyzer 2000.

Although the amplification determination process (step S103) is executed by the CPU 231a of the nucleic acid amplification analyzer 2000 in the above embodiment, the CPU 231a also may transmit the amplification curve MV generated in the step S102 to the quality control terminal device 3000, so that the amplification determination process (step S103) may be executed by the quality control terminal device 3000.

### Other Embodiments

The embodiments described above are intended to facilitate understanding of the present invention, and are not intended to limit interpretation of the present invention. The present invention can be modified and improved (for example, combining each embodiment, omitting a part of the configuration of each embodiment) without departing from the spirit thereof, and the present invention also includes equivalents thereof.

### EXPLANATION OF REFERENCE NUMBERS

1: Nucleic acid amplification analysis system, 210: Pretreatment unit, 220: Measurement unit (nucleic acid amplification unit and detection unit), 230: Data processing unit (control unit), 2000: Nucleic acid amplification analyzer, 3000: Quality control terminal device (determination device).

## Claims

1. A method for determining a success or failure of a nucleic acid amplification comprising:
obtaining a measurement value corresponding to the amount of a nucleic acid amplified by a nucleic acid amplification reaction of a measurement sample containing the nucleic acid in a state in which the nucleic acid amplification reaction has reached a saturation state; and
determining the success or failure of a nucleic acid amplification reaction based on the acquired measurement value.

2. The method for determining the success or failure of the nucleic acid amplification according to claim 1, further comprising:
determining the success or failure of the nucleic acid amplification reaction by comparing the obtained measurement value with measurement values obtained in a state where a nucleic acid amplification reaction has reached a saturation state for other measurement samples in the past.

3. The method for determining the success or failure of the nucleic acid amplification according to claim 2, wherein,
in the determination, the success or failure of the nucleic acid amplification is determined by comparing the obtained measurement value with the measurement values obtained in the state where the nucleic acid amplification reaction has reached the saturation state for other measurement samples immediately before.

4. The method for determining the success or failure of the nucleic acid amplification according to claim 2 or 3, wherein
the method for determining the success or failure of the nucleic acid amplification is executed in a device for determining the success or failure of the nucleic acid amplification comprising a plurality of detection units for detecting a measurement value corresponding to the amount of the nucleic acid; and
the determination is realized by comparing the measurement values obtained by the respective plurality of detection units;
with the measurement values obtained in the state in which the nucleic acid amplification reaction has reached the saturation state for other measurement samples in the past.

5. The method for determining the success or failure of the nucleic acid amplification according to claim 2, wherein
the measurement sample to be determined and the other measurement samples in the past are nucleic acids amplified using a nucleic acid amplification reagent of the same production lot.

6. The method for determining the success or failure of the nucleic acid amplification according to any one of claims 1 to 5, wherein
the determination comprises evaluating a state of a nucleic acid amplification reagent used for the amplification of the nucleic acid.

7. The method for determining the success or failure of the nucleic acid amplification according to claim 6, wherein
evaluating the state of the nucleic acid amplification reagent comprises evaluating at least one of a stirred state and a thawed state of the nucleic acid amplification reagent.

8. The method for determining the success or failure of the nucleic acid amplification according to any one of claims 1 to 7, wherein
the measurement value is obtained at a predetermined time in the state in which the nucleic acid amplification reaction has reached the saturation state.

9. The method for determining the success or failure of the nucleic acid amplification according to any one of claims 1 to 8, further comprising:
obtaining the time at which a substantial increase in the amount of the nucleic acid begins, or the number of amplifications at the time;
wherein the determination comprises determining the success or failure of the nucleic acid amplification reaction based on the measurement value, and the time or the number of amplifications at the time.

10. The method for determining the success or failure of the nucleic acid amplification according to claim 9, further comprising:
obtaining information on the amount of the nucleic acid contained in the measurement sample before amplification based on the time at which the substantial increase in the amount of nucleic acid begins or the number of amplifications at the time.

11. The method for determining the success or failure of the nucleic acid amplification according to any one of claims 1 to 10, further comprising:
outputting information on the success or failure of the nucleic acid amplification reaction.

12. The method for determining the success or failure of the nucleic acid amplification according to 11, wherein
the information regarding the success or failure of the nucleic acid amplification reaction comprises a message prompting a measurement operator to confirm the state of the nucleic acid amplification reagent for amplifying the nucleic acid when the determination result of the success or failure of the nucleic acid amplification reaction is failure.

13. A device for determining a success or failure of a nucleic acid amplification comprising:
a nucleic acid amplification unit configured to amplify a nucleic acid contained in a measurement sample;
a detection unit configured to detect the nucleic acid amplification by the nucleic acid amplification unit, and output a measurement value; and
a control unit configured to
control the nucleic acid amplification unit and the detection unit,
obtain the measurement value corresponding to the amplified amount of the nucleic acid in a state in which a nucleic acid amplification reaction has reached a saturation state based on the outputted measurement value from the detection unit, and
determine the success or failure of the nucleic acid amplification reaction based on the obtained measurement value.

14. A nucleic acid amplification analysis system comprising:
a nucleic acid amplification analyzer having a nucleic acid amplification unit that amplifies nucleic acid contained in a measurement sample, and a detection unit that detects nucleic acid amplification by the nucleic acid amplification unit and outputs a measurement value;
a determination unit, communicably connected to the nucleic acid amplification analyzer through a network, configured to
obtain the measurement value corresponding to the amplified amount of the nucleic acid in a state in which the nucleic acid amplification reaction has reached a saturation state based on the output from the detection unit, and
determine a success or failure of the nucleic acid amplification reaction based on the obtained measurement value.

15. The nucleic acid amplification analysis system according to claim 14, wherein
the control unit is configured to determine the success or failure of the nucleic acid amplification reaction by comparing the obtained measurement value with measurement values in the state where the nucleic acid amplification reaction has reached the saturation state for other measurement samples in the past.
